# EUROPÄISCHE PATENTSCHRIFT

(11) **EP 2 222 886 B1**
(45) Veröffentlichungstag und Bekanntmachung des Hinweises auf die Patenterteilung: **29.02.2012**
(21) Anmeldenummer: 08863918.2
(22) Anmeldetag: 12.12.2008
(51) Int. Cl.: C23C 8/02, C23C 8/10, B22F 3/24, C04B 41/50, C04B 41/87

(54) **IMPLANTAT UND VERFAHREN ZU DESSEN HERSTELLUNG INSBESONDERE DESSEN OBERFLÄCHENMODIFIKATION**
IMPLANT AND PROCESS FOR PRODUCING IT, IN PARTICULAR MODIFICATION OF ITS SURFACE
IMPLANT ET SON PROCÉDÉ DE FABRICATION, EN PARTICULIER MODIFICATION DE SA SURFACE

(30) Priorität: 20.12.2007 CH 19822007
(43) Veröffentlichungstag der Anmeldung: 01.09.2010
(73) Patentinhaber: THOMMEN MEDICAL AG, 4437 Waldenburg (CH)
(72) Erfinder: ORTEGA CRUZ, Luis, Alfonso, 2545 Selzach (CH); SCHLOTTIG, Falko, 4414 Füllinsdorf (CH)
(74) Vertreter: Bremi, Tobias Hans
(86) Internationale Anmeldenummer: PCT/CH2008/000527
(87) Internationale Veröffentlichungsnummer: WO 2009/079805

(56) Entgegenhaltungen:
- WO-A-2006/131010
- DE-A1- 19 858 501
- US-A1- 2001 036 530

## Beschreibung

### TECHNISCHES GEBIET

Die vorliegende Erfindung betrifft ein Verfahren zur Herstellung eines an der Oberfläche wenigstens im Knochen und/oder Gewebe ausgesetzten Bereich strukturierten keramischen Implantats. Des weiteren werden Implantate beschrieben und beansprucht, welche unter Zuhilfenahme dieses Verfahrens an der Oberfläche mit einer topologischen Struktur versehen wurden.

### STAND DER TECHNIK

Es gibt eine Vielzahl von Implantaten, welche seit langem als Ersatzmaterial oder Aufbaumaterial in den menschlichen Körper eingesetzt werden. Im Bereich der so genannten Knochenimplantate, das heisst derjenigen Implantate, welche in wenigstens teilweise den Knochen eingesetzt werden, so beispielsweise Gelenksimplantate, Wirbelsäulenimplantate, Dentalimplantate, haben sich in der Hauptsache Implantate aus Metall oder aus Keramik durchgesetzt. Je nach erforderlichen Materialeigenschaften und Vorzügen des Patienten wird das eine oder das andere Material verwendet.

Damit Implantate in den Knochen und/oder das angrenzende Gewebe fest einwachsen, ist es üblich, die Oberfläche der Implantate entweder zu modifizieren oder auf die Oberfläche der Implantate eine Beschichtung aufzubringen. Einerseits geht es dabei darum, die Oberfläche mit einer topologischen Struktur zu versehen, beispielsweise einer Rauigkeit in einem bestimmten mikroskopischen Bereich, damit das Gewebe respektive der Knochen in der Lage ist, eine Verankerung zum Implantat aufzubauen. Andererseits geht es darum, die Oberfläche mit das einwachsen begünstigenden respektive aktivierenden Beschichtungen zu versehen, beispielsweise mit Wirksubstanzen, welche das angrenzende Gewebe dazu stimulieren, die Verankerung respektive den Prozess des Einwachsens beschleunigt durchzuführen.

Im Zusammenhang mit Implantaten gibt es verschiedene Verfahren zur Herstellung einer strukturierten, das heisst rauen Oberfläche. Auf der einen Seite kann dies durch eine Strahlbehandlung, beispielsweise durch Strahlen mit Sand einer bestimmten Korngrösse (mesh) erfolgen. Auf der anderen Seite kann dies durch eine chemische Behandlung, beispielsweise durch eine Behandlung, welche die Oberfläche teilweise angreift und auflöst respektive modifiziert, erfolgen. Für den Fall der metallischen Implantate beschreibt beispielsweise die EP 0 388 576 ein Verfahren, in welchem das Implantat, gegebenenfalls nach vorgängiger Behandlung durch Sandstrahlen, konzentrierter Säure ausgesetzt wird. Für den Fall der keramischen Implantate beschreibt beispielsweise die WO 00/37121 ein Verfahren, in welchem die Oberfläche in einer Lauge behandelt wird.

Generell zeigt es sich, dass je nach verwendeten Basismaterial (Metall/Keramik) unterschiedliche Verfahren zur Behandlung der Oberfläche zur Erzeugung einer Strukturierung bekannt sind und diese unterschiedlichen Verfahren auch zu unterschiedlichen Strukturen an den Oberflächen der Implantate Anlass geben.

Die WO 2006/0131010 beschreibt ein keramisches Implantat, namentlich ein Dentalimplantat, mit einer strukturierten, resp. porösen Oberfläche zum wenigstens teilweisen Einsetzen in einen Knochen. Die besonders vorteilhafte poröse Oberfläche ist dabei wenigstens bereichsweise salzschmelzenmodifiziert. Die hervorragenden Osteointegrationseigenschaften können in einem Verfahren bewirkt werden, bei welchem nach dessen Formgebung, gegebenenfalls nach vorgängiger abtragender Oberflächenmodifikation, wenigstens in den Knochen und/oder Weichgewebe ausgesetzten Bereichen diese Oberfläche in einer Salzschmelze oberflächenmodifiziert wird.

### DARSTELLUNG DER ERFINDUNG

Der Erfindung liegt demnach die Aufgabe zugrunde, ein alternatives Verfahren zur Strukturierung von Oberflächen von keramischen Implantaten zur Verfügung zu stellen. Es soll sich dabei um ein möglichst vielseitiges Verfahren handeln, welches durch Einstellung der Verfahrensparameter in der Lage ist, unterschiedlichste Topologien zu erzeugen. Es handelt sich also um eine Verbesserung eines Verfahrens zur Herstellung eines an der Oberfläche wenigstens im Knochen und/oder Gewebe ausgesetzten Bereich strukturierten keramischen Implantats, sowie um entsprechende Implantate, insbesondere Dental-Implantate.

Die Lösung dieser Aufgabe wird dadurch erreicht, dass das keramische Implantat nach dessen Formgebung und ggf. bereits einer Sinterung im zu strukturierenden Bereich
I. an der Oberfläche zum entsprechenden Metall reduziert wird;
II. anschliessend die im wesentlichen metallische Oberfläche einer strukturierenden Oberflächenbehandlung unterzogen wird;
III. die strukturierte Oberfläche wieder zurück oxidiert wird.

Einer der wesentlichen Aspekte der Erfindung besteht mit anderen Worten darin, dass erkannt wurde, dass es unerwarteter Weise problemlos möglich ist, ein keramisches Implantat zunächst im Oberflächenbereich in die metallische Form zu reduzieren (Oxidationszustand 0), dass es anschliessend möglich ist, sämtliche aus dem Bereich der metallischen Implantate bekannten Verfahren zur Strukturierung der Oberfläche an dieser obersten metallischen Schicht durchzuführen, und anschliessend die so strukturierte metallische Oberflächenschicht wieder zurück oxidiert werden kann. Auf diese Weise können die vielen bekannten Verfahren zur Strukturierung einer metallischen Oberfläche im Schritt II. eingesetzt und beispielsweise aus der Literatur übernommen werden. Anschliessend entsteht aber infolge des Schrittes III. eine ähnliche aber bei vielen Verfahrensbedingungen nicht genau die gleiche Struktur wie bei der Behandlung eines metallischen Implantates nur mit dem Schritt II., das heisst es werden neuartige Strukturierungen an der Oberfläche zur Verfügung gestellt.

Ein wesentlicher Vorteil des vorgeschlagenen Verfahrens besteht darin, dass infolge des grossen Wissens über die Strukturierung von metallischen Oberflächen dieses Wissen wenigstens teilweise auf die Strukturierung von Keramik-Implantaten übertragen werden kann. Es kann so beinahe im beliebigen Rahmen eine angepasste Strukturierung der Oberfläche eines Keramik-Implantats erzeugt werden, in Abhängigkeit des gewählten Verfahrens im Schritt II. (gegebenenfalls in Kombination mit einer vorgängigen oder nachträglichen Strukturierung) sowie dessen Einstellung der Parameter.

Ein wesentlicher Vorteil des vorgeschlagenen Verfahrens besteht darin, dass die Oberfläche des keramischen Implantats unter Verwendung des Materials des Implantates erzeugt wird (gewissermassen autochthone Struktur) und nicht unter Zuhilfenahme von Beschichtungsverfahren. Letztere führen in der Regel zu Problemen mit der Schichthaftung, welche beim hier vorgeschlagenen neuartigen Verfahren vollständig ausgeschlossen werden können, weil beispielsweise im Schritt I. keine klassische Schicht mit einer glatten Grenzfläche zum keramischen Material ausgebildet wird sondern vielmehr eine stark verzahnte Oberflächenmodifikation, welche sich an der Festigkeit und der Porosität des bearbeiteten Materials orientiert und entsprechend auch in jedem Verfahrensstadium einen innigen Verbund gewährleistet.

Die Keramikoberfläche (typischerweise ZrO₂ und Mischungen von ZrO₂ und Al₂O₃) wird also zunächst in einer definierten Umgebung bis zum Metall reduziert. Die erhaltene Metalloberfläche wird durch Ätzprozesse oder andere Verfahren strukturiert. Die strukturierte metallische Oberfläche wird in definierter Umgebung oxidiert. Daraus resultiert die angestrebte strukturierte keramische Implantatoberfläche.

Eine erste Ausführungsform des vorgeschlagenen Verfahrens ist dadurch gekennzeichnet, dass das keramische Implantat vor respektive während dessen Formgebung und/oder Sinterung bereits einer Strukturierung der Oberfläche unterzogen worden ist (z.B. Strukturierung der Oberfläche des Grünlings oder auch Strukturierung respektive Belegung der Form für die Formgebung mit Platzhaltern).

Alternativ oder zusätzlich ist es auch möglich, dass das keramische Implantat nach dessen Formgebung und/oder Sinterung aber vor der Behandlung in den Schritten I.-III. bereits einer Strukturierung der Oberfläche unterzogen worden ist.

Die vorgängige Strukturierung der Oberfläche des keramischen Implantats kann mit anderen Worten zum Beispiel durch Oberflächenstrukturierung des Grünlings vor dem Sintern erfolgen. Sie kann aber auch durch Verwendung einer Form beim Schritt der Formgebung, welche Form auf der Innenseite strukturiert ist oder Platzhalter aufweist erfolgen. Sie kann gleichermassen durch Oberflächenstrukturierung des gesinterten Keramik-Implantats erfolgen (dieses kann auch bereits einer zusätzlichen Schleifbehandlung oder einem anderen Verfahren unterzogen worden sein). Diese genannten Strukturierung in der Oberfläche können alternativ oder gemeinsam angewendet werden. Generell kann es sich bei der Strukturierung bei diesem Verfahren vorzugsweise um eine mechanische Behandlung wie Strahlbehandlung und/oder um eine chemische Behandlung wie Säurebehandlung, Laugenbehandlung, Salzschmelzenbehandlung handeln. Die Bedingungen bei der chemischen Behandlung sind dabei analog, wie sie weiter unten für den Schritt II. beschrieben werden.

Vorzugsweise wird, sowohl bei der Strukturierung der Oberfläche des Grünlings als auch bei der Oberfläche des gesinterten Implantats entweder ein einzelnes Strahlmittel oder aber bevorzugt eine Mischung aus zwei Strahlmitteln mit unterschiedlicher Korngrössenverteilung und/oder aus unterschiedlichem Material verwendet. So kann es sich gemäss einer weiteren bevorzugten Ausführungsform bei der Strahlbehandlung zum Beispiel um eine Strahlbehandlung mit gleichzeitiger und/oder nacheinander durchgeführter Behandlung mit zwei Strahlmitteln unterschiedlicher mittlerer Korngrösse handeln. So vorzugsweise als Mischung mit einem Anteil eines Strahlmittels einer groben Korngrösse im Bereich von 80-180 mesh und einem Anteil einer geringen Korngrösse im Bereich von 300-450 mesh, wobei es sich beim Strahlmittel um Sand und/oder organisches Material wie Obstkerne handeln kann. Besonders bevorzugt ist in diesem Zusammenhang feiner Sand und grobe Obstkerne. Die Verwendung einer Mischung von zwei Strahlmitteln mit unterschiedlicher Korngrössenverteilung führt zu Ausbildung einer bimodalen Porositätverteilung, das heisst einer Makrostruktur, welche von einer Mikrostruktur überlagert ist. Eine solche Oberflächenstruktur ist in Bezug auf das Einwachsverhalten in Kombination mit der nachgeschalteten Behandlung in den Schritten L-III. von grossem Vorteil.

Eine weitere bevorzugte Ausführungsform ist dadurch gekennzeichnet, dass im Schritt I. unter reduzierender Gasatmosphäre, bevorzugt unter Wasserstoffatmosphäre, reduziert wird. Diese Reduktion unter Gasatmosphäre erfolgt vorzugsweise bei einer erhöhten Temperatur, namentlich typischerweise oberhalb von 500°C, bevorzugt oberhalb von 800 °C, insbesondere bevorzugt im Bereich von 1000°C-1400°C. Des weiteren erfolgt die Behandlung unter Reduzieren der Gasatmosphäre bevorzugtermassen über eine Dauer von wenigstens 10 Minuten, bevorzugt wenigstens 30 Minuten, insbesondere bevorzugt im Bereich von 40 Minuten-20 Stunden.

Alternativ oder zusätzlich ist es möglich, dass im Schritt I. unter Verwendung von Graphit reduziert wird, vorzugsweise im Rahmen des Sinterns, insbesondere bevorzugt als isostatisches Heisspressen (HIP) in Anwesenheit von Graphit. Das Graphit wird, insbesondere beim HIP-Verfahren, vorzugsweise als Reduktionsmittel bei einer Temperatur oberhalb von 500°C, bevorzugt oberhalb von 1000 °C, insbesondere bevorzugt im Bereich von 1200°C-1800°C eingesetzt. Die Reduktion unter Verwendung von Graphit findet vorzugsweise über eine Dauer von wenigstens 10 Minuten statt, bevorzugt wenigstens 30 Minuten, insbesondere bevorzugt im Bereich von 40 Minuten-20 Stunden. Bevorzugtermassen wird bei einem Druck oberhalb von 200 bar, vorzugsweise oberhalb von 500 bar, insbesondere bevorzugt im Bereich von 800 bar-1500 bar gearbeitet.

Gemäss einer weiteren Ausführungsform wird im Schritt II. die im wesentlichen metallische Oberfläche im zu strukturierenden Bereich einer Säurebehandlung, insbesondere unter Zuhilfenahme von konzentrierter Säure, unterzogen. Die Säurebehandlung erfolgt bevorzugtermassen unter Verwendung einer Mischung von starken Säuren, so beispielsweise unter Verwendung einer Mischung von Salzsäure und/oder Schwefelsäure und/oder Salpetersäure. Bevorzugt wird dabei beispielsweise eine Mischung aus 50% Salzsäure (circa 30 prozentig) und 25% Schwefelsäure (circa 95-97 prozentig) eingesetzt. Generell wird bei der Säurebehandlung vorzugsweise bei einer Temperatur oberhalb von 80°C, insbesondere bevorzugt bei einer Temperatur oberhalb von 100°C, so beispielsweise bei 120°C, gearbeitet. Bevorzugtermassen erfolgt die Säurebehandlung über eine Zeitdauer von wenigstens 60 Minuten, insbesondere bevorzugt von wenigstens 100 Minuten, vorzugsweise im Bereich von 110-400 Minuten.

Alternativ oder zusätzlich ist es möglich, im Schritt II. die im wesentlichen metallische Oberfläche im zu strukturierenden Bereich einer Behandlung in einer Salzschmelze zu unterziehen. Dies bevorzugtermassen unter Zuhilfenahme von Alkali- und/oder Erdalkali-Chloriden und/oder -Hydroxiden. Bevorzugt wird mit einer Mischung aus Lithiumhydroxid und Natriumhydroxid (vorzugsweise im Verhältnis 1:2 - 2:1) gearbeitet. Die Behandlung in einer Salzschmelze erfolgt bevorzugtermassen bei einer Temperatur oberhalb von 80°C, insbesondere bevorzugt bei einer Temperatur oberhalb von 100°C. Die Behandlung in der Salzschmelze erfolgt vorzugsweise über eine Zeitdauer von wenigstens 20 Minuten, insbesondere bevorzugt von wenigstens 40 Minuten, vorzugsweise im Bereich von 80-400 Minuten.

Eine weitere bevorzugte Ausfiihrungsform der Erfindung ist dadurch gekennzeichnet, dass im Schritt III. das an der Oberfläche strukturierte Implantat unter einer oxidierenden Gasatmosphäre, bevorzugtermassen unter Luft, bei einer Temperatur oberhalb von 800°C, vorzugsweise oberhalb von 1000°C, insbesondere bevorzugt im Bereich von 1000°C-1500°C gehalten wird. Die Behandlung unter dieser oxidierenden Atmosphäre erfolgt bevorzugtermassen über eine Dauer von wenigstens 60 Minuten, vorzugsweise über eine Dauer von wenigstens 100 Minuten, insbesondere bevorzugt über eine Dauer im Bereich von 120 - 600 Minuten. Auch andere Oxidationsverfahren können eingesetzt werden, so beispielsweise Säureoxidation, Plasmaoxidation oder ähnliche, welche dieser Verfahren für eine bestimmte Art der Keramik besonders gut geeignet sind, lässt sich durch Versuche vom Fachmann ermitteln.

Gemäss einer bevorzugten Ausführungsform findet das Verfahren Anwendung zur Strukturierung eines keramischen Implantats auf Metalloxid-Basis, bevorzugt auf Aluminiumoxid-Basis und/oder Zirkonoxid-Basis und/oder Silikonnitrid-Basis handelt, insbesondere bevorzugt auf Yttrium-stabilisierten Tetragonal-zirkonpolykristallen (Y-TZP).

Ohne dieses zu beanspruchen beschreibt des weiteren die vorliegende Erfindung ein keramisches Implantat, insbesondere Dental-Implantat, hergestellt nach einem Verfahren, wie es oben dargestellt wurde. Es handelt es sich dabei um ein Implantat auf Zirkonoxid-Basis, insbesondere bevorzugt basierend auf Yttrium-stabilisierten Tetragonal-zirkonpolykristallen (Y-TZP).

Des weiteren betrifft die vorliegende Erfindung die Verwendung eines Verfahrens, wie es oben beschrieben wurde, zur Strukturierung der Oberfläche eines Implantats, bevorzugt eines Dental-Implantats wenigstens im den Knochen und/oder dem Zahnfleisch ausgesetzten Bereich.

### KURZE ERLÄUTERUNG DER FIGUREN

Die Erfindung soll nachfolgend anhand von Ausführungsbeispielen im Zusammenhang mit den Figuren näher erläutert werden. Es zeigen sämtliche Figuren rasterelektronenmikroskopische Aufnahmen (SEM) der Oberflächen von Beispielen, wie sie unten im Detail beschrieben werden. Gerät: Rasterelektronenmikroskop Leica Cambridge S-360. Alle Aufnahmen wurden bei einer Hochspannung von 20kV im Hochvakuum durchgeführt. Die Proben wurden vorgängig im PVC-Verfahren mit ca. 10nm Gold besputtert.

### WEGE ZUR AUSFÜHRUNG DER ERFINDUNG

Es wurde eine Vielzahl von verschiedenen Oberflächenstrukturierung in vorgenommen, um die Vielseitigkeit der unterschiedlichen Strukturen zu dokumentieren. Im wesentlichen wurde für die Reduktion mit Wasserstoff sowie mit Graphit gearbeitet, und für die Behandlung der metallischen Oberfläche wurde mit Säure sowie mit Salzschmelze gearbeitet. Teilweise wurde der Grünling bereits strukturiert respektive die Form für das Sintern an der Oberfläche so strukturiert, dass der resultierende Formkörper eine Oberflächenstrukturierung aufwies. Die Parameter wie Temperatur, Behandlungsdauer etc. wurden variiert, um zu dokumentieren, wie vielfältig die resultierende Struktur eingestellt werden kann. Wo nichts anderes spezifisch angegeben wird, wird bei der Strahlbehandlung als Strahlmittel Aluminiumoxid Al₂O₃ mit der angegebenen Teilchengrösse verwendet.

Die Rauheitsmessungen der Oberfläche des Implantates wurden jeweils am Gewindegrund gemessen, Vorrichtungen, Methoden und Messparameter: Confocal microscopy 3 dimensional. White light microscopy CLA (Chromatic Light Aberration) ALTI SURF 500. Optical probe range: 300µm. Lateral resolution: < 2µm. Z Auflösung 10 nm. Gaussian filter mit cut off = 32µm, Sz, Sds and Ssc Parameter sind definiert nach EUR15178N report. ISO 13565 für RK Parameter. Alle Messwerte in µm.

### Vorgehen 1: Reduktion der Keramikoberfläche in Wasserstoff Ätzen mit der metallisierten Oberfläche mit Säure

### Beispiel 1:

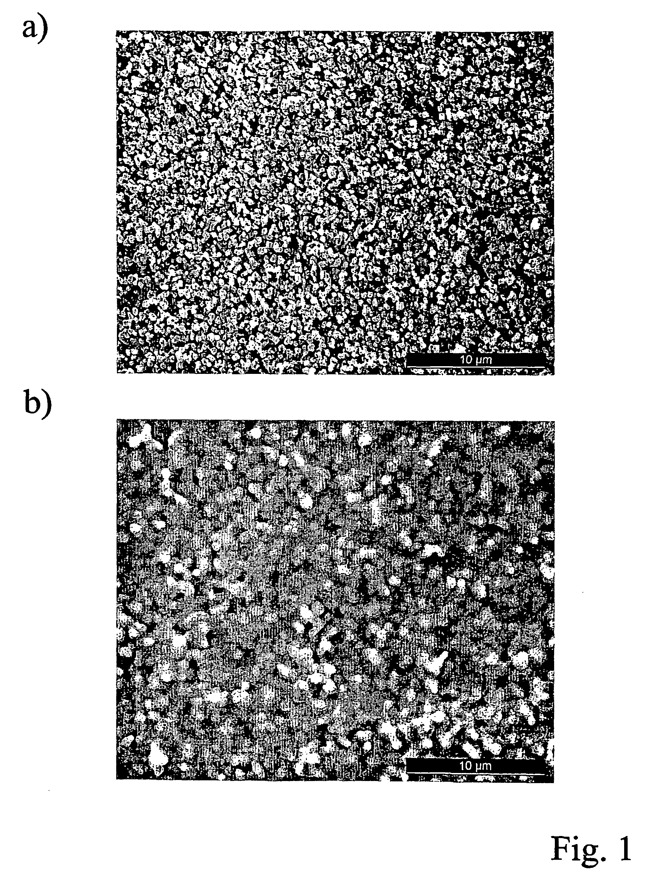

Ein Grünling in Form eines zylindrischen Zahnimplantates mit 10 mm Länge und 4 mm Durchmesser wurde aus yttriumstabilisiertem Zirkonoxidpulver gespritzt (Y-TZP, CIM-Verfahren). Der Grünling wurde nicht sandgestrahlt. Nach dem Spritzen und Sintern wurde die Oberfläche des Teils in H2 Atmosphäre bei 1200°C 60 min reduziert (Schritt I.). Die so metallisierte Oberfläche wurde mit einer Säuremischung (50% HCl (32%-ig) / 25% H₂SO₄ (95 - 97%-ig)) bei 140°C für 120 min geätzt (Schritt II). Die erhaltene strukturierte Oberfläche des Implantates vor der Oxidation ist in Figur 1a dargestellt. Die so strukturierte Oberfläche wurde bei 1'350°C, 140 min oxidiert (Schritt III.). Die erhaltene strukturierte Oberfläche des oxidierten Implantates ist in Figur 1b dargestellt. Die gemessenen Rauhigkeiten waren wie folgt:

| Sa | Sq | St | Sk | Rt | Rq | Ra |
|---|---|---|---|---|---|---|
| 0.665 | 0.805 | 5.21 | 0.399 | 1.62 | 0.191 | 0.052. |

### Beispiel 2:

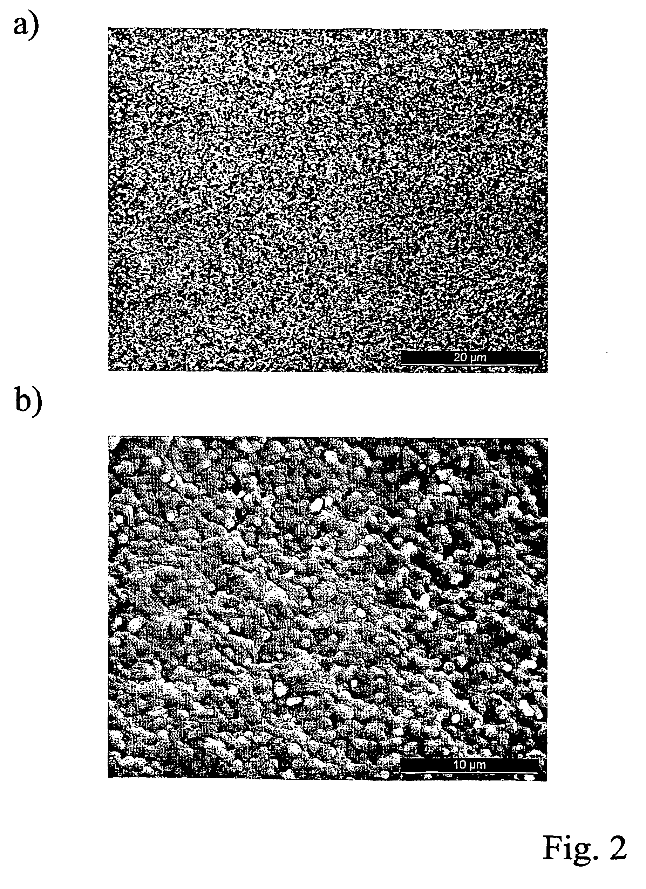

Ein Grünling in Form eines zylindrischen Zahnimplantates mit 10 mm Länge und 4 mm Durchmesser wurde aus yttriumstabilisiertem Zirkonoxidpulver gespritzt (Y-TZP, CIM-Verfahren). Der Grünling wurde nicht sandgestrahlt. Nach dem Spritzen und Sintern wurde die Oberfläche des Teils in H2 Atmosphäre bei 1200°C 60 min reduziert. Die so metallisierte Oberfläche wurde mit einer Säuremischung (50% HCL (32%-ig) / 25% H₂SO₄ (95 - 97%-ig)) bei 140°C für 120 min geätzt. Die so strukturierte Oberfläche wurde bei 1'200°C, 140 min oxidiert. Die erhaltene strukturierte Oberfläche des Implantates ist in Abbildungen 2a und 2b dargestellt. Die gemessenen Rauhigkeiten waren wie folgt:

| Sa | Sq | St | Sk | Rt | Rq | Ra |
|---|---|---|---|---|---|---|
| 0.665 | 0.805 | 5.21 | 0.399 | 1.62 | 0.191 | 0.052. |

### Beispiel 3:

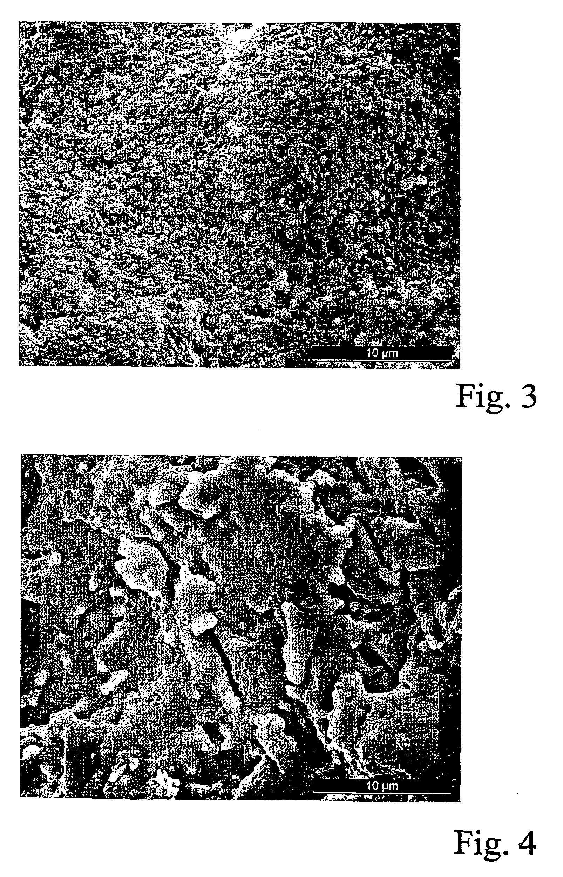

Ein Grünling in Form eines zylindrischen Zahnimplantates mit 10 mm Länge und 4 mm Durchmesser wurde aus yttriumstabilisiertem Zirkonoxidpulver gespritzt (Y-TZP, CIM-Verfahren). Der Grünling wurde nicht sandgestrahlt. Dabei wurde aber das Spritzwerkzeug mittels erodieren so modifiziert, dass der Grünling nach dem Spritzen eine Makrostruktur aufweist. Danach wurde die Oberfläche des gespritzten Grünlings in H2 Atmosphäre bei 1200°C 60 min reduziert. Die so metallisierte Oberfläche wurde mit einer Säuremischung (50% HCL (32%-ig) / 25% H₂SO₄ (95 - 97%-ig)) bei 110°C für 300 min geätzt. Die so strukturierte Oberfläche wurde bei 1'200°C, 140 min oxidiert. Die erhaltene strukturierte Oberfläche des Implantates ist in Figur 3 dargestellt. Die gemessenen Rauhigkeiten waren wie folgt:

| Sa | Sq | St | Sk | Rt | Rq | Ra |
|---|---|---|---|---|---|---|
| 2.52 | 3.31 | 28 | 1.34 | 7.01 | 0.72 | 0.567. |

### Beispiel 4:

Ein Grünling in Form eines zylindrischen Zahnimplantates mit 10 mm Länge und 4 mm Durchmesser wurde aus yttriumstabilisiertem Zirkonoxidpulver gespritzt (Y-TZP, CIM-Verfahren). Dabei wurde das Spritzwerkzeug mittels erodieren so modifiziert, dass der Grünling nach dem Spritzen eine Makrostruktur aufweist. Der Grünkörper wurde zusätzlich mit Al₂O₃ gestrahlt, mit 120 Mesh Teilchengrösse. Danach wurde die Oberfläche des gespritzten und gesinterten Teils in H2 Atmosphäre bei 1200°C 60 min reduziert. Die so metallisierte Oberfläche wurde mit einer Säuremischung (50% HCL (32%-ig) / 25% H₂SO₄ (95 - 97%-ig)) bei 110°C für 300 min geätzt. Die so strukturierte Oberfläche wurde bei 1'200°C, 140 min oxidiert. Die erhaltene strukturierte Oberfläche des Implantates ist in Figur 4 dargestellt. Die gemessenen Rauhigkeiten waren wie folgt:

| Sa | Sq | St | Sk | Rt | Rq | Ra |
|---|---|---|---|---|---|---|
| 4.13 | 5.12 | 35.8 | 0.99 | 5.35 | 0.534 | 0.429. |

### Beispiel 5:

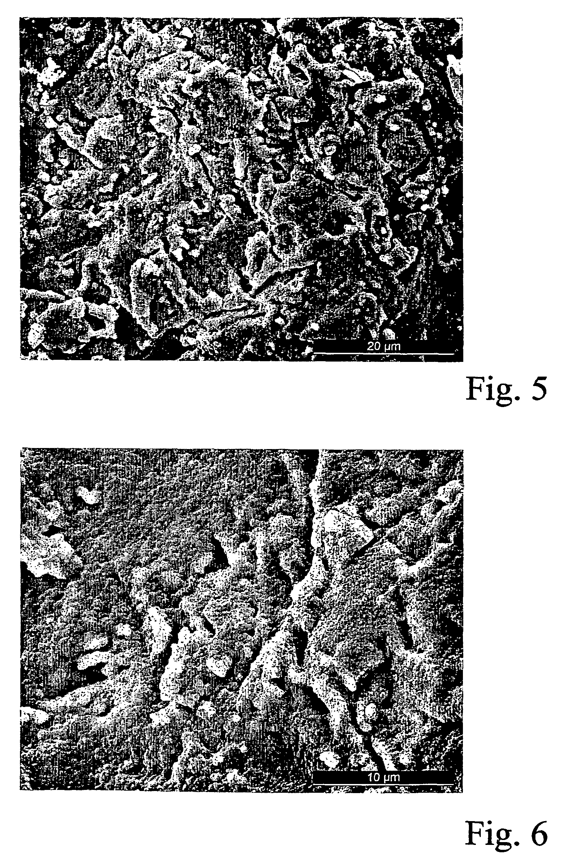

Ein Grünling in Form eines zylindrischen Zahnimplantates mit 10 mm Länge und 4 mm Durchmesser wurde aus yttriumstabilisiertem Zirkonoxidpulver gespritzt (Y-TZP, CIM-Verfahren). Dabei wurde das Spritzwerkzeug mittels erodieren so modifiziert, dass der Grünling nach dem Spritzen eine Makrostruktur aufweist. Der Grünkörper wurde zusätzlich Al₂O₃ gestrahlt, mit 360 Mesh Teilchengrösse. Danach wurde die Oberfläche des gespritzten und gesinterten Teils in H2 Atmosphäre bei 1200°C 60 min reduziert. Die so metallisierte Oberfläche wurde mit einer Säuremischung (50% HCL (32%-ig) / 25% H₂SO₄ (95 - 97%-ig)) bei 110°C Für 120 min geätzt. Die so strukturierte Oberfläche wurde bei 1'200°C, 140 min oxidiert. Die erhaltene strukturierte Oberfläche des Implantates ist in Figur 5 dargestellt. Die gemessenen Rauhigkeiten waren wie folgt:

| Sa | Sq | St | Sk | Rt | Rq | Ra |
|---|---|---|---|---|---|---|
| 1.86 | 2.54 | 33.8 | 1.06 | 3.95 | 0.518 | 0.423. |

### Beispiel 6:

Ein Grünling in Form eines zylindrischen Zahnimplantates mit 10 mm Länge und 4 mm Durchmesser wurde aus yttriumstabilisiertem Zirkonoxidpulver gespritzt (Y-TZP, CIM-Verfahren). Dabei wurde das Spritzwerkzeug mittels erodieren so modifiziert, dass der Grünling nach dem Spritzen eine Makrostruktur aufweist. Der Grünkörper wurde zusätzlich Al₂O₃ gestrahlt, mit 360 Mesh Teilchengrösse. Danach wurde die Oberfläche des gespritzten und gesinterten Teils in H2 Atmosphäre bei 1200°C 60 min reduziert. Die so metallisierte Oberfläche wurde mit einer Säuremischung (50% HCL (32%-ig) / 25% H₂SO₄ (95 - 97%-ig)) bei 110°C für 60 min geätzt. Die so strukturierte Oberfläche wurde bei 1'200°C, 140 min oxidiert. Die erhaltene strukturierte Oberfläche des Implantates ist in Figur 6 dargestellt. Die gemessenen Rauhigkeiten waren wie folgt:

| Sa | Sq | St | Sk | Rt | Rq | Ra |
|---|---|---|---|---|---|---|
| 1.86 | 2.54 | 33.8 | 1.06 | 3.95 | 0.518 | 0.423. |

### Beispiel 7:

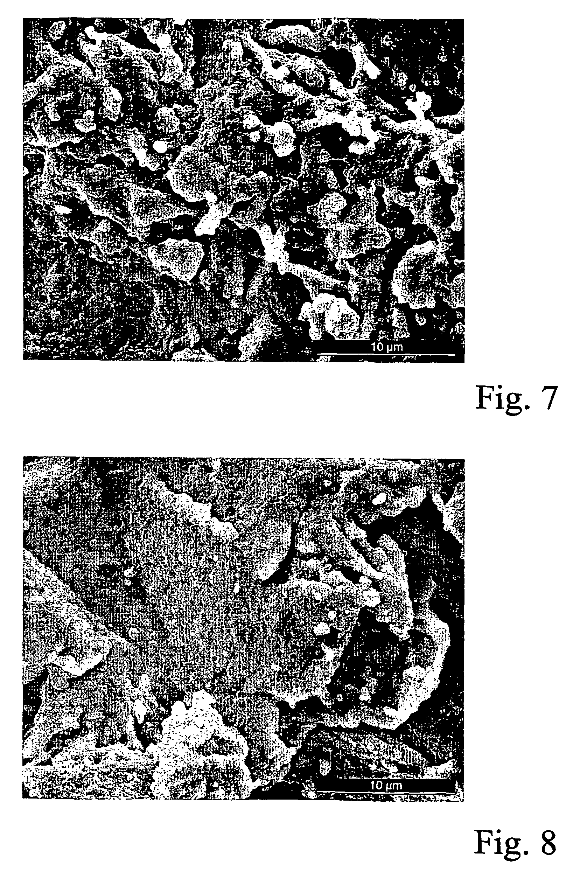

Ein Grünling in Form eines zylindrischen Zahnimplantates mit 10 mm Länge und 4 mm Durchmesser wurde aus yttriumstabilisiertem Zirkonoxidpulver gespritzt (Y-TZP, CIM-Verfahren). Dabei wurde das Spritzwerkzeug mittels erodieren so modifiziert, dass der Grünling nach dem Spritzen eine Makrostruktur aufweist. Der Grünkörper wurde zusätzlich Al₂O₃ gestrahlt, mit 360 Mesh Teilchengrösse. Danach wurde die Oberfläche des gespritzten und gesinterten Teils in H2 Atmosphäre bei 1200°C 60 min reduziert. Die so metallisierte Oberfläche wurde mit einer Säuremischung (50% HCL (32%-ig) / 25% H₂SO₄ (95 - 97%-ig)) bei 110°C für 300 min geätzt. Die so strukturierte Oberfläche wurde bei 1'200°C, 140 min oxidiert. Die erhaltene strukturierte Oberfläche des Implantates ist in Figur 7 dargestellt. Die gemessenen Rauhigkeiten waren wie folgt:

| Sa | Sq | St | Sk | Rt | Rq | Ra |
|---|---|---|---|---|---|---|
| 1.86 | 2.54 | 33.8 | 1.06 | 3.95 | 0.518 | 0.423. |

### Beispiel 8:

Ein Grünling in Form eines zylindrischen Zahnimplantates mit 10 mm Länge und 4 mm Durchmesser wurde aus yttriumstabilisiertem Zirkonoxidpulver gespritzt (Y-TZP, CIM-Verfahren). Dabei wurde das Spritzwerkzeug mittels erodieren so modifiziert, dass der Grünling nach dem Spritzen eine Makrostruktur aufweist. Der Grünkörper wurde zusätzlich Al₂O₃ gestrahlt, mit 360 Mesh Teilchengrösse. Danach wurde die Oberfläche des gespritzten und gesinterten Teils in H2 Atmosphäre bei 1200°C 60 min reduziert. Die so metallisierte Oberfläche wurde mit einer Säuremischung (50% HCL (32%-ig) / 25% H₂SO₄ (95 - 97%-ig)) bei 110°C für 120 min geätzt. Die so strukturierte Oberfläche wurde bei 1'200°C, 140 min oxidiert. Die erhaltene strukturierte Oberfläche des Implantates ist in Figur 8 dargestellt. Die gemessenen Rauhigkeiten waren wie folgt:

| Sa | Sq | St | Sk | Rt | Rq | Ra |
|---|---|---|---|---|---|---|
| 4.13 | 5.12 | 35.8 | 0.99 | 5.35 | 0.534 | 0.429. |

### Beispiel 9:

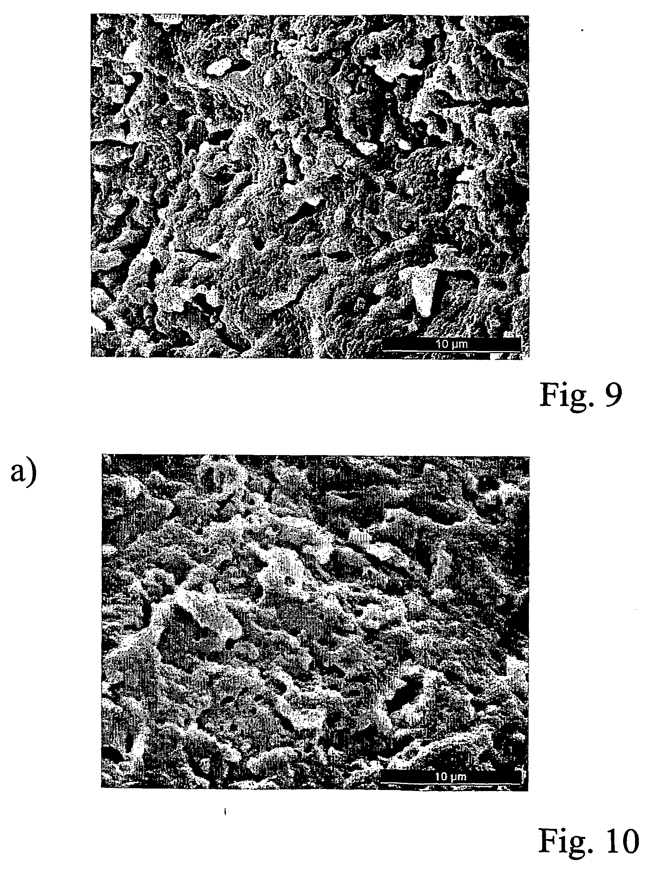

Ein Grünling in Form eines zylindrischen Zahnimplantates mit 10 mm Länge und 4 mm Durchmesser wurde aus yttriumstabilisiertem Zirkonoxidpulver gespritzt (Y-TZP, CIM-Verfahren). Dabei wurde das Spritzwerkzeug mittels erodieren so modifiziert, dass der Grünling nach dem Spritzen eine Makrostruktur aufweist. Der Grünkörper wurde zusätzlich Al₂O₃ gestrahlt, mit einer Mischung aus 120 und 360 Mesh Grösse. Danach wurde die Oberfläche des gespritzten und gesinterten Teils in H2 Atmosphäre bei 1200°C 60 min reduziert. Die so metallisierte Oberfläche wurde mit einer Säuremischung (50% HCL (32%-ig) / 25% H₂SO₄ (95 - 97%-ig)) bei 110°C für 60 min geätzt. Die so strukturierte Oberfläche wurde bei 1'200°C, 140 min oxidiert. Die erhaltene strukturierte Oberfläche des Implantates ist in Figur 9 dargestellt. Die gemessenen Rauhigkeiten waren wie folgt:

| Sa | Sq | St | Sk | Rt | Rq | Ra |
|---|---|---|---|---|---|---|
| 4.13 | 5.12 | 35.8 | 0.99 | 5.35 | 0.534 | 0.429. |

### Vorgehen 2 Reduktion der Keramikoberfläche mit Graphit während des HIP Prozesses, Ätzen der metallisierten Oberfläche mit einer Salzschmelze

### Beispiel 10:

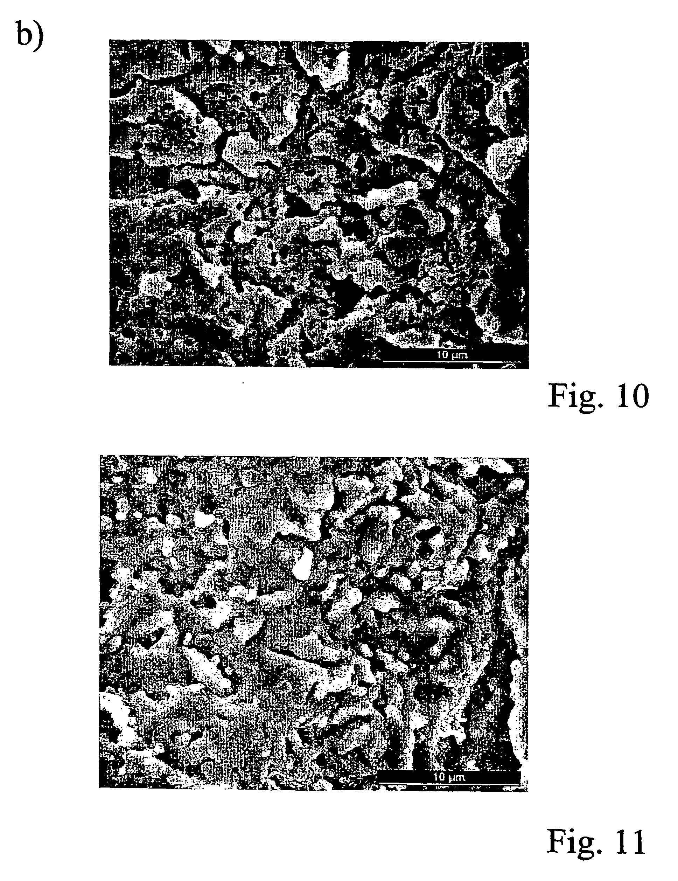

Ein Grünling in Form eines zylindrischen Zahnimplantates mit 10 mm Länge und 4 mm Durchmesser wurde aus yttriumstabilisiertem Zirkonoxidpulver gespritzt (Y-TZP, CIM-Verfahren). Dabei wurde das Spritzwerkzeug mittels erodieren so modifiziert, dass der Grünling nach dem Spritzen eine Makrostruktur aufweist. Der Grünkörper wurde zusätzlich Al₂O₃ gestrahlt, mit einer Mischung aus 120 und 360 Mesh Grösse. Danach wurde die Oberfläche des gespritzten und gesinterten Teils mittels Graphit im HIP Prozess bei 1400°C, 1000 bar und 60 min reduziert. Die so metallisierte Oberfläche wurde mit einer Salzschmelze (50% LiOH / 50% NaOH bei 200°C für 30 Stunden geätzt. Die erhaltene strukturierte Oberfläche des noch nicht oxidierten Implantates ist in Figur 10a dargestellt. Die so strukturierte Oberfläche wurde bei 1'000°C, 140 min oxidiert. Die erhaltene strukturierte Oberfläche des Implantates ist in Figur 10b dargestellt. Die gemessenen Rauhigkeiten waren wie folgt:

| Sa | Sq | St | Sk | Rt | Rq | Ra |
|---|---|---|---|---|---|---|
| 3.69 | 4.66 | 35 | 1.04 | 4.62 | 0.529 | 0.425. |

### Vorgehen 3 Reduktion der Keramikoberfläche mit Graphit während des HIP Prozesses, Ätzen der metallisierten Oberfläche mit Säure

### Beispiel 11:

Ein Grünling in Form eines zylindrischen Zahnimplantates mit 10 mm Länge und 4 mm Durchmesser wurde aus yttriumstabilisiertem Zirkonoxidpulver gespritzt (Y-TZP, CIM-Verfahren). Dabei wurde das Spritzwerkzeug mittels erodieren so modifiziert, dass der Grünling nach dem Spritzen eine Makrostruktur aufweist. Der Grünkörper wurde zusätzlich Al₂O₃ gestrahlt, mit einer Mischung aus 120 und 360 Mesh Grösse. Danach wurde die Oberfläche des gespritzten und gesinterten Teils mittels Graphit im HIP Prozess bei 1400°C, 1000 bar und 60 min reduziert. Die so metallisierte Oberfläche wurde mit einer Säuremischung (50% HCL (32%-ig) / 25% H2SO4 (95 - 97%-ig)) bei 110°C für 30 min geätzt. Die so strukturierte Oberfläche wurde bei 1'200°C, 140 min oxidiert. Die erhaltene strukturierte Oberfläche des Implantates ist in Figur 11 dargestellt. Die gemessenen Rauhigkeiten waren wie folgt:

| Sa | Sq | St | Sk | Rt | Rq | Ra |
|---|---|---|---|---|---|---|
| 4.13 | 5.12 | 35.8 | 0.99 | 5.35 | 0.534 | 0.429. |

### Beispiel 12:

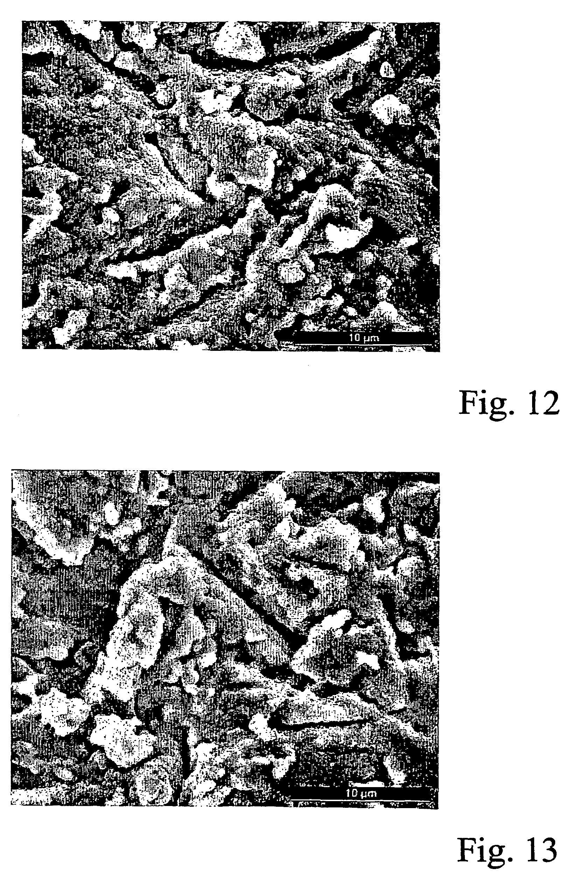

Ein Grünling in Form eines zylindrischen Zahnimplantates mit 10 mm Länge und 4 mm Durchmesser wurde aus yttriumstabilisiertem Zirkonoxidpulver gespritzt (Y-TZP, CIM-Verfahren). Dabei wurde das Spritzwerkzeug mittels erodieren so modifiziert, dass der Grünling nach dem Spritzen eine Makrostruktur aufweist. Der Grünkörper wurde zusätzlich Al₂O₃ gestrahlt, mit 360 Mesh Teilchengrösse. Danach wurde die Oberfläche des gespritzten und gesinterten Teils mittels Graphit im HIP Prozess bei 1400°C, 1000 bar und 60 min reduziert. Die so metallisierte Oberfläche wurde mit einer Säuremischung (50% HCL (32%-ig) / 25% H2S04 (95 - 97%-ig)) bei 110°C für 120 min geätzt. Die so strukturierte Oberfläche wurde bei 1'200°C, 140 min oxidiert. Die erhaltene strukturierte Oberfläche des Implantates ist in Figur 12 dargestellt Die gemessenen Rauhigkeiten waren wie folgt:

| Sa | Sq | St | Sk | Rt | Rq | Ra |
|---|---|---|---|---|---|---|
| 2.35 | 308 | 24.7 | 1.23 | 4.36 | 0.586 | 0.483. |

### Beispiel 13:

Ein Grünling in Form eines zylindrischen Zahnimplantates mit 10 mm Länge und 4 mm Durchmesser wurde aus yttriumstabilisiertem Zirkonoxidpulver gespritzt (Y-TZP, CIM-Verfahren). Dabei wurde das Spritzwerkzeug mittels erodieren so modifiziert, dass der Grünling nach dem Spritzen eine Makrostruktur aufweist. Der Grünkörper wurde zusätzlich Al₂O₃ gestrahlt, mit 360 Mesh Teilchengrösse. Danach wurde die Oberfläche des gespritzten und gesinterten Teils mittels Graphit im HIP Prozess bei 1400°C, 1000 bar und 60 min reduziert. Die so metallisierte Oberfläche wurde mit einer Säuremischung (50% HCL (32%-ig) / 25% H2SO4 (95 - 97%-ig)) bei 110°C für 30 min geätzt. Die so strukturierte Oberfläche wurde bei 1'200°C, 140 min oxidiert. Die erhaltene strukturierte Oberfläche des Implantates ist in Figur 13 dargestellt. Die gemessenen Rauhigkeiten waren wie folgt:

| Sa | Sq | St | Sk | Rt | Rq | Ra |
|---|---|---|---|---|---|---|
| 2.35 | 308 | 24.7 | 1.23 | 4.36 | 0.586 | 0.483. |

### Beispiel 14:

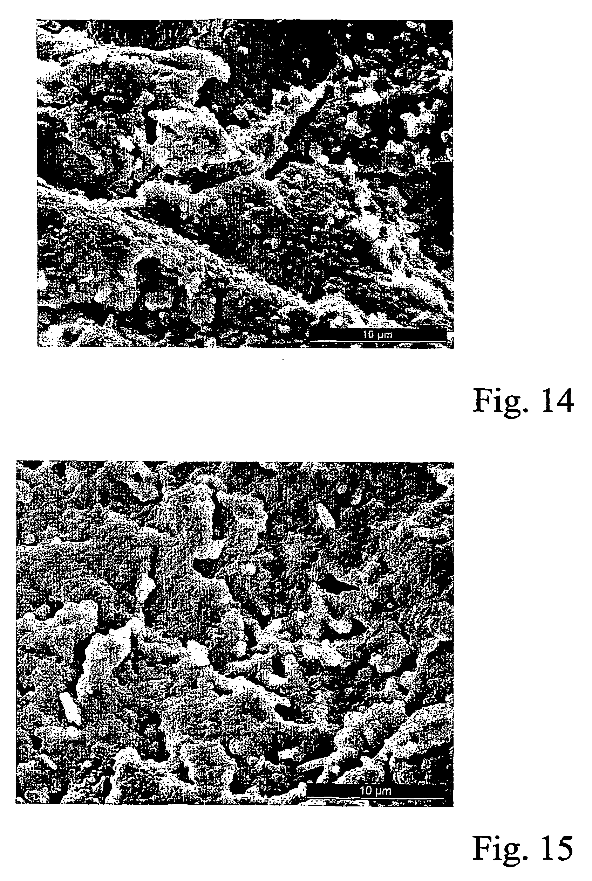

Ein Grünling in Form eines zylindrischen Zahnimplantates mit 10 mm Länge und 4 mm Durchmesser wurde aus yttriumstabilisiertem Zirkonoxidpulver gespritzt (Y-TZP, CIM-Verfahren). Dabei wurde das Spritzwerkzeug mittels erodieren so modifiziert, dass der Grünling nach dem Spritzen eine Makrostruktur aufweist. Der Grünkörper wurde zusätzlich Al₂O₃ gestrahlt, mit 120 Mesh Teilchengrösse. Danach wurde die Oberfläche des gespritzten und gesinterten Teils mittels Graphit im HIP Prozess bei 1400°C, 1000 bar und 60 min reduziert. Die so metallisierte Oberfläche wurde mit einer Säuremischung (50% HCL (32%-ig) / 25% H2SO4 (95 - 97%-ig)) bei 110°C für 120min geätzt. Die so strukturierte Oberfläche wurde bei 1'200°C, 140 min oxidiert. Die erhaltene strukturierte Oberfläche des Implantates ist in Figur 14 dargestellt. Die gemessenen Rauhigkeiten waren wie folgt:

| Sa | Sq | St | Sk | Rt | Rq | Ra |
|---|---|---|---|---|---|---|
| 4.13 | 5.12 | 35.8 | 0.99 | 5.35 | 0.534 | 0.429. |

### Beispiel 15:

Ein Grünling in Form eines zylindrischen Zahnimplantates mit 10 mm Länge und 4 mm Durchmesser wurde aus yttriumstabilisiertem Zirkonoxidpulver gespritzt (Y-TZP, CIM-Verfahren). Dabei wurde das Spritzwerkzeug mittels erodieren so modifiziert, dass der Grünling nach dem Spritzen eine Makrostruktur aufweist. Der Grünkörper wurde zusätzlich Al₂O₃ gestrahlt, mit einer Mischung aus 120 und 360 Mesh Grösse. Danach wurde die Oberfläche des gespritzten und gesinterten Teils mittels Graphit im HIP Prozess bei 1400°C, 1000 bar und 60 min reduziert. Die so metallisierte Oberfläche wurde mit einer Säuremischung (50% HCL (32%-ig) / 25% H2SO4 (95 - 97%-ig)) bei 110°C für 300min geätzt. Die so strukturierte Oberfläche wurde bei 1'200°C, 140 min oxidiert. Die erhaltene strukturierte Oberfläche des Implantates ist in Figur 15 dargestellt. Die gemessenen Rauhigkeiten waren wie folgt:

| Sa | Sq | St | Sk | Rt | Rq | Ra |
|---|---|---|---|---|---|---|
| 4.13 | 5.12 | 35.8 | 0.99 | 5.35 | 0.534 | 0.429. |

### Beispiel 16:

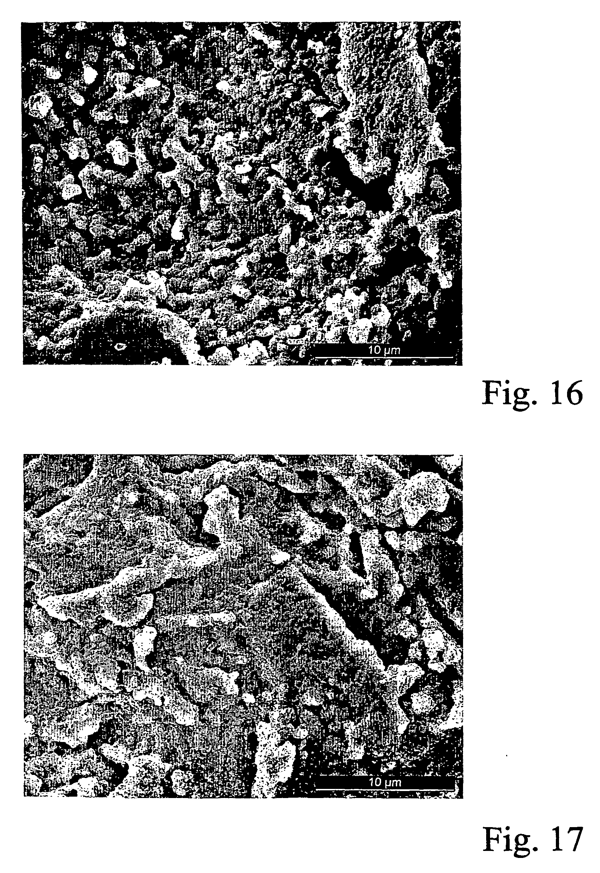

Ein Grünling in Form eines zylindrischen Zahnimplantates mit 10 mm Länge und 4 mm Durchmesser wurde aus yttriumstabilisiertem Zirkonoxidpulver gespritzt (Y-TZP, CIM-Verfahren). Dabei wurde das Spritzwerkzeug mittels erodieren so modifiziert, dass der Grünling nach dem Spritzen eine Makrostruktur aufweist. Der Grünkörper wurde zusätzlich Al₂O₃ gestrahlt, mit 120 Mesh Teilchengrösse. Danach wurde die Oberfläche des gespritzten und gesinterten Teils mittels Graphit im HIP Prozess bei 1400°C, 1000 bar und 60 min reduziert. Die so metallisierte Oberfläche wurde mit einer Säuremischung (50% HCL (32%-ig) / 25% H2SO4 (95 - 97%-ig)) bei 110°C für 300min geätzt. Die so strukturierte Oberfläche wurde bei 1'200°C, 140 min oxidiert. Die erhaltene strukturierte Oberfläche des Implantates ist in Figur 16 dargestellt. Die gemessenen Rauhigkeiten waren wie folgt:

| Sa | Sq | St | Sk | Rt | Rq | Ra |
|---|---|---|---|---|---|---|
| 4.13 | 5.12 | 35.8 | 0.99 | 5.35 | 0.534 | 0.429. |

### Beispiel 17:

Ein Grünling in Form eines zylindrischen Zahnimplantates mit 10 mm Länge und 4 mm Durchmesser wurde aus yttriumstabilisiertem Zirkonoxidpulver gespritzt (Y-TZP, CIM-Verfahren). Dabei wurde das Spritzwerkzeug mittels erodieren so modifiziert, dass der Grünling nach dem Spritzen eine Makrostruktur aufweist. Der Grünkörper wurde zusätzlich Al₂O₃ gestrahlt, mit 360 Mesh Teilchengrösse. Danach wurde die Oberfläche des gespritzten und gesinterten Teils mittels Graphit im HIP Prozess bei 1400°C, 1000 bar und 60 min reduziert. Die so metallisierte Oberfläche wurde mit einer Säuremischung (50% HCL (32%-ig) / 25% H2SO4 (95 - 97%-ig)) bei 110°C für 210min geätzt. Die so strukturierte Oberfläche wurde bei 1'200°C, 140 min oxidiert. Die erhaltene strukturierte Oberfläche des Implantates ist in Figur 17 dargestellt. Die gemessenen Rauhigkeiten waren wie folgt:

| Sa | Sq | St | Sk | Rt | Rq | Ra |
|---|---|---|---|---|---|---|
| 2.35 | 308 | 24.7 | 1.23 | 4.36 | 0.586 | 0.483. |

### Beispiel 18:

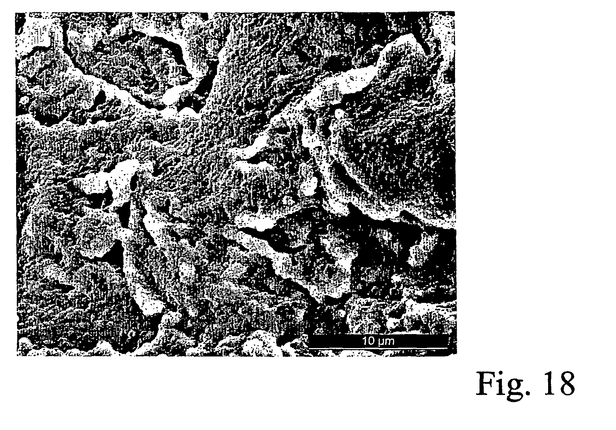

Ein Grünling in Form eines zylindrischen Zahnimplantates mit 10 mm Länge und 4 mm Durchmesser wurde aus yttriumstabilisiertem Zirkonoxidpulver gespritzt (Y-TZP, CIM-Verfahren). Dabei wurde das Spritzwerkzeug mittels erodieren so modifiziert, dass der Grünling nach dem Spritzen eine Makrostruktur aufweist. Der Grünkörper wurde zusätzlich Al₂O₃ gestrahlt, mit 360 Mesh Teilchengrösse. Danach wurde die Oberfläche des gespritzten und gesinterten Teils mittels Graphit im HIP Prozess bei 1400°C, 1000 bar und 60 min reduziert. Die so metallisierte Oberfläche wurde mit einer Säuremischung (50% HCL (32%-ig) / 25% H2SO4 (95 - 97%-ig)) bei 110°C für 300 min geätzt. Die so strukturierte Oberfläche wurde bei 1'200°C, 140 min oxidiert. Die erhaltene strukturierte Oberfläche des Implantates ist in Figur 18 dargestellt. Die gemessenen Rauhigkeiten waren wie folgt:

| Sa | Sq | St | Sk | Rt | Rq | Ra |
|---|---|---|---|---|---|---|
| 2.35 | 308 | 24.7 | 1.23 | 4.36 | 0.586 | 0.483. |

## Patentansprüche

1. Verfahren zur Herstellung eines an der Oberfläche wenigstens im Knochen und/oder Gewebe ausgesetzten Bereich strukturierten keramischen Implantats, **dadurch gekennzeichnet, dass**
das keramische Implantat nach dessen Formgebung im zu strukturierenden Bereich
I. an der Oberfläche zum entsprechenden Metall reduziert wird;
II. anschliessend die im wesentlichen metallische Oberfläche einer strukturierenden Oberflächenbehandlung unterzogen wird;
III. die strukturierte Oberfläche wieder zurück oxidiert wird.

2. Verfahren nach Anspruch 1, **dadurch gekennzeichnet, dass** das keramische Implantat vor dem Schritt I. gesintert wird.

3. Verfahren nach Anspruch 1 oder 2, **dadurch gekennzeichnet, dass** das keramische Implantat, bevorzugt vor dessen Formgebung und ggf. Sinterung und/oder nach dessen Formgebung und ggf. Sinterung aber vor der Behandlung in den Schritten I.-III., bereits einer Strukturierung der Oberfläche unterzogen wird.

4. Verfahren nach Anspruch 2 oder 3, **dadurch gekennzeichnet, dass** die vorgängige Strukturierung der Oberfläche des keramischen Implantats durch Oberflächenstrukturierung des Grünlings vor dem Sintern, und/oder durch Verwendung einer Form beim Schritt der Formgebung, welche auf der Innenseite strukturiert ist oder Platzhalter aufweist, und/oder durch Oberflächenstrukturierung des gesinterten Keramik-Implantats.

5. Verfahren nach Anspruch 4, **dadurch gekennzeichnet, dass** die vorgängige Strukturierung der Oberfläche des keramischen Implantats durch Oberflächenstrukturierung des Grünlings vor dem Sintern, und/oder durch Verwendung einer Form beim Schritt der Formgebung, welche auf der Innenseite strukturiert ist oder Platzhalter aufweist, und/oder durch Oberflächenstrukturierung des gesinterten Keramik-Implantats, wobei die Strukturierung durch eine mechanische Behandlung wie Strahlbehandlung und/oder durch eine chemische Behandlung wie Säurebehandlung, Laugenbehandlung, Salzschmelzenbehandlung, erfolgt.

6. Verfahren nach Anspruch 4 oder 5, **dadurch gekennzeichnet, dass** es sich bei der Strahlbehandlung um eine Strahlbehandlung mit gleichzeitiger und/oder nacheinander durchgeführter Behandlung mit zwei Strahlmitteln unterschiedlicher mittlerer Korngrösse handelt, vorzugsweise mit zwei Strahlmitteln unterschiedlicher mittlerer Korngrösse mit einem Anteil einer groben Korngrösse im Bereich von 80-180 mesh und einem Anteil einer geringen Korngrösse im Bereich von 300-450 mesh, wobei es sich beim Strahlmittel um Sand und/oder organisches Material wie Obstkerne handeln kann.

7. Verfahren nach einem der vorhergehenden Ansprüche, **dadurch gekennzeichnet, dass** im Schritt I. unter reduzierender Gasatmosphäre, bevorzugt unter Wasserstoffatmosphäre, reduziert wird, vorzugsweise bei einer Temperatur oberhalb von 500°C, und/oder vorzugsweise über eine Dauer von wenigstens 10 Minuten, bevorzugt wenigstens 30 Minuten.

8. Verfahren nach einem der vorhergehenden Ansprüche, **dadurch gekennzeichnet, dass** im Schritt I. unter reduzierender Gasatmosphäre, bevorzugt unter Wasserstoffatmosphäre, reduziert wird, bei einer Temperatur oberhalb von 800 °C, bevorzugt im Bereich von 1000°C-1400°C, und/oder vorzugsweise über eine Dauer im Bereich von 40 Minuten-20 Stunden.

9. Verfahren nach einem der vorhergehenden Ansprüche, **dadurch gekennzeichnet, dass** im Schritt I. unter Verwendung von Graphit reduziert wird, vorzugsweise im Rahmen des Sinterns, insbesondere bevorzugt als isostatisches Heisspressen (HIP), wobei das Graphit vorzugsweise als Reduktionsmittel bei einer Temperatur oberhalb von 500°C, bevorzugt oberhalb von 1000 °C, insbesondere bevorzugt im Bereich von 1200°C-1800°C, und/oder vorzugsweise über eine Dauer von wenigstens 10 Minuten, bevorzugt wenigstens 30 Minuten, insbesondere bevorzugt im Bereich von 40 Minuten-20 Stunden und/oder bei einem Druck oberhalb von 200 bar, vorzugsweise oberhalb von 500 bar, insbesondere bevorzugt im Bereich von 800 bar-1500 bar eingesetzt wird.

10. Verfahren nach einem der vorhergehenden Ansprüche, **dadurch gekennzeichnet, dass** im Schritt II. die im wesentlichen metallische Oberfläche im zu strukturierenden Bereich einer Säurebehandlung, insbesondere unter Zuhilfenahme von konzentrierter Säure, insbesondere bevorzugt einer Mischung von Salzsäure und/oder Schwefelsäure und/oder Salpetersäure, unterzogen wird, wobei vorzugsweise bei einer Temperatur oberhalb von 80°C, insbesondere bevorzugt bei einer Temperatur oberhalb von 100°C, bevorzugtermassen über eine Zeitdauer von wenigstens 60 Minuten, insbesondere bevorzugt von wenigstens 100 Minuten, vorzugsweise im Bereich von 110-400 Minuten, mit der Säure behandelt wird.

11. Verfahren nach einem der vorhergehenden Ansprüche, **dadurch gekennzeichnet, dass** im Schritt II. die im wesentlichen metallische Oberfläche im zu strukturierenden Bereich einer Behandlung in einer Salzschmelze unterzogen wird, insbesondere unter Zuhilfenahme von Alkali- und/oder Erdalkali-Chloriden und/oder Hydroxiden, insbesondere bevorzugt bei einer Temperatur oberhalb von 80°C, insbesondere bevorzugt bei einer Temperatur oberhalb von 100°C, wobei bevorzugtermassen über eine Zeitdauer von wenigstens 20 Minuten, insbesondere bevorzugt von wenigstens 40 Minuten, vorzugsweise im Bereich von 80-400 Minuten in der Salzschmelze behandelt wird.

12. Verfahren nach einem der vorhergehenden Ansprüche, **dadurch gekennzeichnet, dass** im Schritt III. das an der Oberfläche strukturierte Implantat unter einer oxidierenden Gasatmosphäre, bevorzugtermassen unter Luft, bei einer Temperatur oberhalb von 800°C, vorzugsweise oberhalb von 1000°C gehalten wird, dies über eine Dauer von wenigstens 60 Minuten, vorzugsweise über eine Dauer von wenigstens 100 Minuten.

13. Verfahren nach einem der vorhergehenden Ansprüche, **dadurch gekennzeichnet, dass** im Schritt III. das an der Oberfläche strukturierte Implantat unter einer oxidierenden Gasatmosphäre, bevorzugtermassen unter Luft, bei einer Temperatur im Bereich von 1000°C-1500°C gehalten wird, dies über eine Dauer im Bereich von 120 - 600 Minuten.

14. Verfahren nach einem der vorhergehenden Ansprüche, **dadurch gekennzeichnet, dass** es sich um ein keramisches Implantat auf Metalloxid-Basis, bevorzugt auf Aluminiumoxid-Basis und/oder Zirkonoxid-Basis und/oder Silikonnitrid-Basis handelt, insbesondere bevorzugt auf Yttrium-stabilisierten Tetragonal-zirkonpolykristallen (Y-TZP).

15. Verwendung eines Verfahrens nach einem der Ansprüche 1-14 zur Strukturierung der Oberfläche eines Implantats, bevorzugt eines Dental-Implantats wenigstens im den Knochen und/oder dem Zahnfleisch ausgesetzten Bereich.

## Claims

1. A process for producing a ceramic implant which is structured on the surface, at least in the area exposed to bone and/or tissue,
**characterized in that**
after it has been shaped, the ceramic implant, in the area to be structured,
I is reduced to the corresponding metal on the surface;
II the substantially metallic surface is then subjected to a structuring surface treatment;
III the structured surface is again reoxidized.

2. The process as claimed in claim 1, **characterized in that** the ceramic implant is sintered before step I.

3. The process as claimed in claim 1 and 2, **characterized in that** the ceramic implant is already subjected to a structuring of the surface before being shaped and possibly sintered and/or after being shaped and possibly sintered, but before being treated in steps I-III.

4. The process as claimed in claim 2 or 3, **characterized in that** the preliminary structuring of the surface of the ceramic implant is done by surface structuring of the green body before sintering, and/or by using a mold in the shaping step, which mold is structured on the inside or has spacers, and/or by surface structuring of the sintered ceramic implant.

5. The process as claimed in claim 4, **characterized in that** the antecedent structuring of the surface of the ceramic implant is effected by surface structuring of the green body before sintering, and/or by application of a mold during molding, which is structured on the inner side or exhibits place holders, and/or by surface structuring of the sintered ceramic implant, **in that** the structuring of the mechanical treatment like blasting and/or by a chemical treatment like acid treatment, base treatment, salt melt treatment.

6. The process as claimed in claim 4 or 5, **characterized in that** the abrasive blasting treatment is an abrasive blasting treatment with simultaneous and/or consecutive treatment with two abrasive blasting agents of different mean grain size, preferably with two blasting agents of intermediate grain size with a proportion of a coarse grain size in the range of 80-180 mesh and a proportion of a small grain size in the range of 300-450 mesh, and the abrasive blasting agent can be sand and/or organic material such as fruit kernels.

7. The process as claimed in one of the preceding claims, **characterized in that** the reduction in step I is carried out under a reducing gas atmosphere, preferably under a hydrogen atmosphere, preferably at a temperature above 500°C, and/or preferably for a duration of at least 10 minutes, preferably at least 30 minutes.

8. The process as claimed in one of the preceding claims, **characterized in that** in step I is carried out under a reducing gas atmosphere, preferably under a hydrogen atmosphere, at a temperature above 800°C, preferably in the range of 1000°C-1400°C, and/or especially for a duration in the range of 40 minutes to 2 hours.

9. The process as claimed in one of the preceding claims, **characterized in that** the reduction in step I is carried out using graphite, preferably in the context of the sintering, particularly preferably as hot isostatic pressing (HIP), wherein the graphite is preferably used as reducing agent at a temperature above 500°C, preferably above 1000°C, particularly preferably in the range of 1200°C-1800°C, and/or preferably for a duration of at least 10 minutes, preferably at least 30 minutes, particularly preferably in the range of 40 minutes to 20 hours, and/or at a pressure above 200 bar, preferably above 500 bar, particularly preferably in the range of 800 bar to 1500 bar.

10. The process as claimed in one of the preceding claims, **characterized in that**, in step II, the substantially metallic surface is exposed, in the area to be structured, to a treatment with acid, in particular with the aid of concentrated acid, particularly preferably a mixture of hydrochloric acid and/or sulfuric acid and/or nitric acid, the treatment with acid being carried out preferably at a temperature above 80°C, particularly preferably at a temperature above 100°C, by preference for a duration of at least 60 minutes, particularly preferably at least 100 minutes, preferably in the range of 110-400 minutes.

11. The process as claimed in one of the preceding claims, **characterized in that**, in step II, the substantially metallic surface is subjected, in the area to be structured, to a treatment in a molten salt, in particular with the aid of alkali metal and/or alkaline earth metal chlorides and/or hydroxides, the treatment in the molten salt being carried out particularly preferably at a temperature above 80°C, particularly preferably at a temperature above 100°C, and by preference for a duration of at least 20 minutes, particularly preferably at least 40 minutes, preferably in the range of 80-400 minutes.

12. The process as claimed in one of the preceding claims, **characterized in that**, in step III, the surface-structured implant is kept under an oxidizing gas atmosphere, preferably air, at a temperature above 800°C, preferably above 1000°C, for a duration of at least 60 minutes, preferably for a duration of at least 100 minutes.

13. The process as claimed in one of the preceding claims, **characterized in that**, in step III, the surface-structured implant is kept under an oxidizing gas atmosphere, preferably air, at a temperature in the range of 1000°C-1500°C over a duration of 120 - 600 minutes.

14. The process as claimed in one of the preceding claims, **characterized in that** the implant is a ceramic implant based on metal oxide, preferably based on alumina and/or based on zirconia and/or based on silicon nitride, particularly preferably on yttria-stabilized tetragonal zirconia polycrystals (Y-TZP).

15. The use of the process as claimed in one of claims 1-14 for structuring the surface of an implant, preferably of a dental implant, at least in the area exposed to the bone and/or the gum.

## Revendications

1. Procédé de fabrication d'un implant céramique dont la surface est structurée au moins dans la partie exposée aux os et/ou aux tissus,
**caractérisé en ce que**
après le moulage de l'implant en céramique, la partie à structurer,
I. est réduite à la surface pour obtenir le métal correspondant,
II. subit ensuite un traitement de structuration de sa surface au moins essentiellement métallique et
III. la surface structurée est de nouveau oxydée.

2. Procédé selon la revendication 1, **caractérisé en ce que** l'implant céramique est fritté avant l'étape I.

3. Procédé selon les revendications 1 ou 2, **caractérisé en ce que** l'implant céramique subit déjà une structuration de sa surface avant son moulage, son frittage éventuel et/ou après son moulage et son frittage éventuel mais avant le traitement des étapes I.-III.

4. Procédé selon les revendications 2 ou 3, **caractérisé en ce que** la structuration préalable de la surface de l'implant céramique s'effectue par structuration de la surface de l'ébauche crue avant le frittage et/ou par recours lors de l'étape de moulage à un moule dont le côté intérieur est structuré ou qui présente des dispositifs de maintien et d'immobilisation et/ou par structuration de la surface de l'implant en céramique frittée.

5. Procédé selon la revendication 4, **caractérisé en ce que** la structuration préalable de la surface de l'implant céramique s'effectue par structuration de la surface de l'ébauche crue avant le frittage et/ou par recours lors de l'étape de moulage à un moule dont le côté intérieur est structuré ou qui présente des dispositifs de maintien et d'immobilisation et/ou par structuration de la surface de l'implant en céramique frittée, la structuration s'effectuant par traitement mécanique, par exemple un traitement par projection et/ou par un traitement chimique, par exemple un traitement à l'acide, un traitement à l'alcali ou un traitement au sel fondu.

6. Procédé selon les revendications 4 ou 5, **caractérisé en ce que** le traitement de projection est un traitement de projection accompagné ou suivi par un traitement à l'aide de deux agents de projection de granulométries moyennes différentes, de préférence de deux agents de projection de grains de tailles moyennes différentes dont une fraction de grains de taille grossière est de l'ordre de 80 à 180 mesh et une fraction de grains de petite taille de l'ordre de 300 à 450 mesh, l'agent de projection pouvant être du sable et/ou un matériau organique, par exemple du grain.

7. Procédé selon l'une des revendications précédentes, **caractérisé en ce qu'**à l'étape I., on réduit dans une atmosphère gazeuse réductrice, de préférence dans une atmosphère d'hydrogène, de préférence à une température supérieure à 500°C et/ou de préférence pendant une durée d'au moins 10 minutes et de préférence d'au moins 30 minutes.

8. Procédé selon l'une des revendications précédentes, **caractérisé en ce qu'**à l'étape I., on dans une atmosphère gazeuse réductrice, de préférence dans une atmosphère d°hydrogène, de préférence à une température supérieure à 800°C et de préférence de l'ordre de 1 000°C à 1 400°C et/ou de préférence pendant une durée de l'ordre de 40 minutes à 20 heures.

9. Procédé selon l'une des revendications précédentes, **caractérisé en ce qu'**à l'étape I., on réduit en recourant à du graphite, de préférence dans le cadre du frittage, de façon particulièrement préférable par pressage isostatique à chaud ("HIP"), le graphite servant de préférence d'agent réducteur étant utilisé à une température supérieure à 500°C, de préférence supérieure à 1 000°C et de façon particulièrement préférable de l'ordre de 1 200°C à 1 800°C, de préférence pendant une durée d'au moins 10 minutes, de préférence d'au moins 30 minutes et de façon particulièrement préférable de l'ordre de 40 minutes à 20 heures et/ou à une pression supérieure à 200 bars, de préférence supérieure à 500 bars et de façon particulièrement préférable de l'ordre de 800 bars à 1 500 bars.

10. Procédé selon l'une des revendications précédentes, **caractérisé en ce qu'**à l'étape II., la surface essentiellement métallique de la partie à structurer subit un traitement à l'acide, de préférence en recourant à de l'acide concentré, de façon particulièrement préférable à un mélange d'acide chlorhydrique, d'acide sulfurique et/ou d'acide nitrique, le traitement à l'acide s'effectuant de préférence à une température supérieure à 80°C, de façon particulièrement préférable à une température supérieure à 100°C et de préférence pendant une durée d'au moins 60 minutes, de façon particulièrement préférable d'au moins 100 minutes et mieux dans la plage de 110 à 400 minutes.

11. Procédé selon l'une des revendications précédentes, **caractérisé en ce qu'**à l'étape II., la surface essentiellement métallique de la partie à structurer est exposée à un sel fondu, en recourant en particulier à des chlorures et/ou hydroxydes de métaux alcalins et/ou de métaux alcalino-terreux, de façon particulièrement préférable à une température supérieure à 80°C, de préférence à une température supérieure à 100°C, le traitement au sel fondu s'effectuant de préférence pendant une durée d'au moins 20 minutes, de façon particulièrement préférable d'au moins 40 minutes et mieux dans la plage de 80 à 400 minutes.

12. Procédé selon l'une des revendications précédentes, **caractérisé en ce que** dans l'étape III., l'implant structuré en surface est maintenu dans une atmosphère gazeuse oxydante, de préférence dans l'air, à une température supérieure à 800°C et de préférence supérieure à 1 000°C pendant une durée d'au moins 60 minutes et de préférence pendant une durée d'au moins 100 minutes.

13. Procédé selon l'une des revendications précédentes, **caractérisé en ce que** dans l'étape III., l'implant structuré en surface est maintenu dans une atmosphère gazeuse oxydante, de préférence dans l'air, à une température dans la plage de 1 000°C à 1 500°C et ce pendant une durée de l'ordre de 120 à 600 minutes.

14. Procédé selon l'une des revendications précédentes, **caractérisé en ce que** l'implant est un implant céramique à base d'oxyde métallique, de préférence à base d'oxyde d'aluminium, à base d'oxyde zirconium et/ou à base de nitrure de silicium, et de façon particulièrement préférable à base de polycristaux tétragonaux de zirconium stabilisé par yttrium (Y-TZP).

15. Utilisation d'un procédé selon l'une des revendications 1 à 14 pour structurer la surface d'au moins la partie exposée à l'os et/ou aux gencives d'un implant, de préférence d'un implant dentaire.
